Europäisches Patentamt

European Patent Office

Office européen des brevets

(19)

(11) EP 1 245 956 A1

(12) EUROPEAN PATENT APPLICATION

(43) Date of publication:
02.10.2002 Bulletin 2002/40

(51) Int Cl.⁷: **G01N 33/531**, G01N 33/53, G01N 33/68

(21) Application number: 01107491.1

(22) Date of filing: 29.03.2001

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Roche Diagnostics GmbH 68305 Mannheim (DE)**

(72) Inventor: **The designation of the inventor has not yet been filed**

(54) **Conjugates of defined stoichiometry**

(57) The invention relates to a process for the production of a biomolecule-linker conjugate of uniform stoichiometry. It especially relates to a conjugate consisting of a biomolecule of a molecular weight between 5 kD and 500 kD and a hydrophilic linker molecule said linker having a molecular weight between 1 and 15 kD and between 4 and 60 charged residues, characterized in that said conjugate comprises at least one biomolecule-linker product of uniform stoichiometry in a pre-selected amount.

Figure 10: Mono Q® chromatograph of a crude BSA-Ru(SK)₄-MH conjugate

EP 1 245 956 A1

**Description**

The invention relates to a process for the production of a biomolecule-linker conjugate of uniform stoichiometry. It especially relates to a conjugate consisting of a biomolecule of a molecular weight between 5 kD and 500 kD and a hydrophilic linker molecule said linker having a molecular weight between 1 and 15 kD and between 4 and 60 charged residues, characterized in that said conjugate comprises at least one biomolecule-linker product of uniform stoichiometry in a pre-selected amount.

Binding assays nowadays are widely used in research settings as well as in clinical routine, especially in diagnosis of an infection monitoring of drugs or for assessing normal or abnormal metabolism.

The use of binding assays dates back about 30 years (Engvall, E. and Perlman, P., Immunochemistry 8 (1971) 871-4; van Weemen, B.K. and Schuures A.H.W.M. (1971), FEBS letters 15, 232). Since then enormous progress has been made and methods for carrying out specific binding assays as well as practical applications thereof have become general knowledge to the skilled artisan. Methods and procedures summarized in related text books are herewith included by reference and only few examples shall be specifically mentioned: "Practice and theory of enzyme immunoassays" by P. Tijssen (1992) Elsevier Science Publishers B.V.; and various editions of "Methods in Enzymology", Colowick S.P., Caplan N.O., Eds., Academic Press, dealing with immunological detection methods, especially volumes 70, 73, 74, 84, 92 and 121.

With the exception of very few homogeneous or precipitation type assays, all these assays require conjugates between a biomolecule, e.g. an antibody, with a second molecule necessary to facilitate the measurement of an analyte under investigation. Well-known examples for this second molecule, e.g., are marker or label groups, like enzymes, radio isotopes, luminophores, fluorphores, etc. or haptens.

Conjugates between a biomolecule and e.g. a marker or hapten group can be synthesized by direct chemical coupling of the two different molecular entities or by using a linker or bridging molecule according to state of the art procedures (e.g. Tijssen or Methods in Enzymology, supra).

Conventional conjugates, however, have clear-cut-limitations. It is a well-known problem that the two or three components of such conjugates, i.e. the biomolecule, the linker (if present), and the marker group may mutually and adversely effect each other. This frequently leads to aggregation or background problems.

Dependent on the coupling or linker chemistry chosen, in many cases e.g. amino acid groups of polypeptide which do not end up to carry a marker group, are also modified. This again leads to unpredictable results and through changes in the physico-chemical properties of the biomolecule to problems, like e.g. instability, aggregation or to non-specific binding in immunoassays.

The various components of such a conjugate, e.g., the biomolecule, the linker molecule, and the marker molecule as well as the different coupling products derived thereof are present in statistical amounts. A mere statistical mixture with an average labeling index is obtained. The average labeling index may be controlled to some extend by adjusting the reaction conditions and ratios of the individual molecular entities chosen.

Such statistical mixtures comprise a lot of different conjugation products in different stoichiometries. Biomolecules completely free of a linker or marker structure, conjugates of a 1:1 stoichiometry, 1:2 stoichiometry (i.e. a biomolecule carrying 2 linker or marker molecules, 1:3 stoichiometry and even higher stoichiometry may be present in such conjugates. It is obvious that not all such products are desired and cause problems e.g. with respect to reproducibility of conjugation. In addition, non-labeled biomolecules interfere with labeled biomolecules. It is also known that "over-labeling", i.e. biomolecules labeled with too many hapten or marker structures is the major reason for problems known as non-specific interactions, e.g. background phenomena.

US 5.958,783 discloses that it is possible to reduce background problems caused by a metal chelate complex as marker group by using a hydrophilic linker entity. However, the conjugates according to US 5,958,783 still represent statistical mixtures of various different biomolecule-linker-marker products. Reproducibility of these statistical conjugates still remains a problem.

WO 96/03423 and WO 96/03651 describe that it is possible to introduce marker groups at predetermined positions during synthesis. This way it is possible to synthesize a peptide-linker (marker) product of uniform stoichiometry. However, the limits of peptide synthesis are in the range of 40 to 50 amino acids corresponding to peptides of a molecular weight below 5 kD (kD = kilo Dalton). Polypeptides of more than 5 kD usually are produced by recombinant expression in appropriate host systems and need to be chemically coupled resulting in a conjugate of statistical compositions. The linkers of WO 96/03423 and WO 96/03657 are below 1 kD and do not contain charged residues.

Large biomolecules, e.g. polypeptides of 40 to 50 amino acids usually comprise more than one reactive group amenable to one of the standard coupling chemistries.

The mode and strategy of chemical coupling can be selected as required. In case the biomolecule is a polypeptide coupling chemistries targeting -SH, $-NH_2$ or $-COO^-$ residues as well as the -OH group of tyrosines, the imidazol group of histidines or the heterocyclic imino groups of tryptophanes are at hand. Several appropriate coupling chemistries are known form the art for each of these functional groups (Aslam M., Dent A., Bioconjugation (1998) Mcmillan Reference Ltd., London, pages 216 to 363).

Whenever more than one reactive group is present on a biomolecule chemical coupling with a linker or marker results in a statistical mixture of different biomolecule-linker or biomolecule-marker products.

Even with the most advanced linkers and coupling chemistries the resulting conjugate is a statistical mixture of many different conjugates and the overall result of such conjugation is highly variable and unpredictable. As a consequence it is very difficult to reproduce the overall properties of such conjugates from lot to lot.

It was therefore the task of the present invention to investigate whether a conjugate may be obtained in a more reproducible and defined manner. It was also investigated whether processes can be found and described how to obtain a conjugate comprising at least one pre-selected biomolecule-linker product of uniform stoichiometry.

It surprisingly has been found that problems known in the art can be overcome by the conjugates according to the present invention, their mode of production and their use in appropriate reagents, kits and procedures.

Disclosed is a process for the production of a conjugate comprising at least one biomolecule-linker product of uniform stoichiometry in a pre-selected amount, said conjugate consisting of a biomolecule of a molecular weight between 5 kD and 500 kD and a hydrophilic linker molecule, said linker having a molecular weight between 1 kD and 15 kD and between 4 and 60 charged residues, characterized in that a) the biomolecule and the linker molecule are covalent coupled to each other, b) the different biomolecule-linker products of uniform stoichiometry are fractionated by chromatography, and c) fractions containing a coupling product of uniform stoichiometry are collected.

The invention also relates to a conjugate consisting of a biomolecule of a molecular weight between 5 kD and 500 kD and a hydrophilic linker molecule said linker having a molecular weight between 1 and 15 kD and between 4 and 60 charged residues, characterized in that said conjugate comprises at least one biomolecule-linker product of uniform stoichiometry in a pre-selected amount.

The conjugates according to the present invention are especially useful for performing a biochemical or immunological assay for detection of an analyte in a sample. The invention therefore also relates to a composition of reagents comprising the described conjugate, a test kit comprising such a reagent composition and an immunoassay based on such a conjugate.

## Detailed description of the invention

A preferred embodiment according to the present invention is a process for the production of a conjugate comprising at least one biomolecule-linker product of uniform stoichiometry in a pre-selected amount, said conjugate consisting of a biomolecule of a molecular weight between 5 kD and 500 kD and a hydrophilic linker molecule, said linker having a molecular weight between 1 kD and 15 kD, between 4 and 60 charged residues, characterized in that

a) the biomolecule and the linker molecule are covalent coupled to each other,

b) the different biomolecule-linker products of uniform stoichiometry are fractionated by chromatography, and

c) fractions containing a coupling product of uniform stoichiometry are collected.

The term "conjugate" is used to describe a reagent comprising at least one coupling product between a biomolecule and a linker molecule which optionally may carry a marker molecule, wherein at least one biomolecule-linker product of uniform stoichiometry is present in a pre-selected amount.

The term "linker" may comprise the linker itself as well as a linker already having bound to it a marker group. At some places the fact that the marker group may already be attached to the linker is additionally indicated by writing linker (marker).

Biomolecule in the sense of the present invention may be any naturally occurring or synthetically produced molecule with a molecular weight between 5 and 500 kD composed of biological molecules like amino acids, nucleotides, nucleosides, lipids, and/or sugars. Preferably, the biomolecule is selected from the group consisting of polypeptides, polysaccharides, and lipopolysaccharides. Preferably the biomolecule has a molecular weight in the range of 10 kD to 500 kD. More preferred the biomolecule has a molecular weight I the range of 15 kD to 400 kD. The biomolecule preferably has at least two reactive groups which are amenable to standard coupling chemistry.

In a preferred embodiment the biomolecule is a polypeptide. Preferably such polypeptide comprises 50 to 5000 amino acids, or more preferred 100 to 4000 amino acids.

The linker according to the present invention has a molecular weight of 1 kD to 15 kD and carries at least four charged residues.

The molecular weight of the linker is at least 1000 D, because then the advantages of the linker become particularly apparent. The molecular weight of the linker is preferably in the range of 1000 to 15,000 D, particularly in the range of 1000 to 12,000 D and most preferably in the range of 1000 to 10,000 D. Also preferred are molecular weight ranges for the linker molecule between 1500 and 15000 D and also between 1500 and 10000 D.

Preferably the linker used to produce the conjugate according to the present invention comprises a peptidic backbone.

In the sense of the present invention the term "charge carrier" means a group which is present mainly in an ionic form at a pH value in the range from 6 to 8. The linker preferably contains 4 to 60, particularly preferred 6 to 50 and most preferred 9 to 40 such charge carriers.

It is preferred that all charge carriers are either positively or negatively charged. It is, however, also possible to use linker molecules which carry both, positively and negatively charged residues. In such case it is preferred that the number of positively charged residues is either higher or lower by at least four as compared to the number of negatively charged residues.

The linker preferably comprises at least four negative charge carrier. Examples of suitable negative charge carriers are phosphate, phosphonate, sulphinate, sulphonate, sulphate and carboxylate groups, carboxylate groups and phosphate groups being most preferred.

It is further preferred, that the linker comprises at least four positively charged residues. Examples of positive charge carriers are amino and mono-substituted or poly-substituted amino groups such as mono, di- or trialkyl amino groups, in which alkyl denotes a straight-chained or branched alkyl residue with 1 to 6 C atoms or a cyclic alkyl residue with 3 to 6 C atoms, guanidinyl groups, e.g. of arginine or positively charged heteroaromatic nitrogen groups, as, e.g. found in histidine. The positive charge carriers preferably are selected from basic amino acids such as lysine or substituted amino acids such as diethyllysine.

The linkers can also contain uncharged hydrophilic groups as an alternative to or in addition to the charge carriers. Preferred examples of uncharged hydrophilic groups are ethylene oxide or polyethylene oxide groups with preferably at least three ethylene oxide units, sulphoxide, sulphone, carboxylic acid amide, carboxylic acid ester, phosphonic acid amide, phosphonic acid ester, phosphoric acid amide, phosphoric acid ester, sulphonic acid amide, sulphonic acid ester, sulphuric acid amide and sulphuric acid ester groups. The amide groups are preferably primary amide groups, particularly preferably carboxylic acid amide residues in amino acid side groups e.g. the amino acids asparagine and glutamine. The esters are preferably derived from hydrophilic alcohols, in particular $C_1$-$C_3$ alcohols or diols or triols.

The linker according to the present invention contains a reactive group Z which is used for chemically coupling the linker to the biomolecule. The group Z is preferably an activated carboxylic acid group such as a carboxylic acid halogenide, a carboxylic acid anhydride, a carboxylic acid hydrazide, a carboxylic acid azide or an active ester e.g. an N-hydroxysuccinimide, a p-nitrophenyl, pentafluorophenyl, imidazolyl or N-hydroxybenzotriazolyl ester, an amine, a maleimide, a thiol, a para-aminobenzoyl group or a photoactivatable group e.g. an azide.

The linker used in the present invention may be a "linear" or a "branched" linker. Linear charged linkers are know from US 5,958,783. Such a linear linker preferably has a chain length of 15- 350 atoms and is an alkylene chain modified by the incorporation of heteroatoms such as amide functions. The linear linker containing the free charge carriers is preferably at least partially composed of aminocarboxylic acids units. Such aminocarboxylic acid units preferably are linked together via peptide bonds. The linker may contain naturally occurring as well as synthetic amino acid units. Preferably, the linker comprises repeatedly the dipeptide β-alanine-glutamic acid. A preferred linker structure comprises between 5 and 70 more preferred between 10 and 60 and most preferred between 15 and 50 times the dipeptide β-alanine-glutamic acid.

The linker in a further preferred embodiment is a branched linker.

The branched linker preferably contains a main chain which contains one or several uncharged hydrophilic groups as mentioned above in particular carboxylic acid amide groups or/and polyethylene glycol groups while there are at least four charge carrier in one or several of the side chains. In this case 1 to 10 charge carriers and in particular 1 to 5 charge carriers can for example be present per side chain.

Alternatively the branched linker can also contain charge carriers in the main chain and uncharged hydrophilic groups in one or several side chains. Furthermore embodiments are also conceivable in which the main chain and the side chains contain uncharged hydrophilic groups as well as charge carriers.

The branched linker has the additional advantage that it can be synthesized to carry several marker groups. Covalent coupling of such a linker (marker) structure brings about the introduction of several marker groups per coupling event. Preferred linker (marker) structures carry two or four marker groups.

The length of the main chain of the branched linker is preferably 7 to 200 atoms, particularly preferably 7 to 100 atoms, in which case the main chain is an alkylene chain modified by the incorporation of heteroatoms e.g. O atoms or amide groups and contains at least one branch site, the side chains formed by the branching site preferably have a length of 4 to 100 atoms. The charge carriers are preferably located in the linker in such a manner that a H atom of an alkylene unit of the main chain or/and in a side chain is replaced by a group containing a charge carrier e. g. $NH_3^+$ or $CO_2^-$ or a guadinyl group.

The branched linker which contains the free charge carriers or/and hydrophilic groups is preferably at least partially composed of aminocarboxylic acid units that are linked together by peptide bonds. In such a linker the branching points can be derived from polyfunctional aminocarboxylic acids which contain at least three functional groups e. g. amino or carboxylate groups such that one functional group is still present after incorporation into the main chain which can be used as the starting point for the synthesis of the side chain. The branches are particularly preferably generated with diaminocarboxylic acids such as lysine, ornithine, hydroxylysine, $\alpha,\beta$-diamino propionic acid etc.

The charge carriers of the branched linker can be preferably derived from free positively or/and negatively charged groups of polyfunctional amino-carboxylic acids which contain a total of at least three charged groups e.g. amino, carboxylate or phosphate groups such that after incorporation into the linker and the concomitant reaction of two of the charged groups, at least one free charge carrier is still present. For example the charge carriers can be derived from trifunctional aminocarboxylic acids which contain (a) an amino group and two carboxylate groups or (b) two amino groups and one carboxylate group. Examples of such trifunctional aminocarboxylic acids are lysine, ornithine, hydroxylysine, $\alpha,\beta$-diamino propionic acid, arginine, aspartic acid and glutamic acid, carboxy glutamic acid and symmetric trifunctional carboxylic acids like those described in EP-A-0 618 192 or US-A-5,519,142. Alternatively one of the carboxylate groups in the trifunctional aminocarboxylic acids (a) can be replaced by a phosphate, sulphonate or sulphate group. An example of such a trifunctional amino acid is phosphoserine.

Alternatively the branched linker can also be composed at least partially of phosphate-sugar units e.g. a DNA backbone without nucleobases or composed of glyco-peptidic structures. Furthermore the linker can also be composed at least partially of saccharide units. In any case the side chain of the linker is preferably situated at a branch of the main chain which is formed by a trifunctional unit and the length of a side chain is at least two of the building blocks used for the synthesis e.g. natural or synthetic amino acids or other components such as ethylene glycol.

According to the present invention it is possible to couple a linker molecule having a molecular weight of 1 to 15 kD to a biomolecule of 5 to 500 kD and to isolate or select coupling products of uniform stoichiometry. The isolated fractions then can be used to couple a second molecule to the first conjugate.

In a preferred embodiment the charged linker molecule which shall be used in the chemical coupling to a biomolecule already carries one or several marker groups. Examples of marker groups are labeling groups and effector groups.

The marker groups already bound to the linker according to the present invention preferably have a molecular weight of less than 20000 D. The smaller the marker group the more difficult is the separation of conjugation products of uniform stoichiometry between a biomolecule and a marker according to the state of the art procedures.

Preferably, the marker group (labeling groups or effector groups) has a molecular weight below 15000 D, more preferred of less than 10000 D. Most preferred the marker group is even smaller than 5000 D. In a preferred embodiment the marker group attached to the linker has a molecular weight of 3000 D or less.

The labeling group can be selected from any detectable known groups, such as dyes, luminescent labeling groups such as chemiluminescent groups e.g. acridinium esters or dioxetanes, or fluorescent dyes e.g. fluorescein, coumarin, rhodamine, oxazine, resorufin, cyanine and derivatives thereof. Other examples of labeling groups are luminescent metal complexes such as ruthenium or europium complexes, enzymes as used for CEDIA (Cloned Enzyme Donor Immunoassay, e.g. EP-A-0 061 888), and radioisotopes.

Effector groups comprise for example one partner of a bioaffine binding pair. While performing an assay the effector group interacts specifically and preferably non-covalent with the other partner of the bioaffine binding pair. Examples of suitable binding partners are hapten or antigen/antibody, biotin or biotin analogues such as aminobiotin, iminobiotin or destheiobiotin/avidin or streptavidin, sugar/lectin, nucleic acid or nucleic acid analogue/complementary nucleic acid, receptor/ligand e.g. steroid hormone receptor/steroid hormone. Preferably, the binding pair member of lower molecular weight is coupled to the linker before the linker (marker) is coupled to the biomolecule. Preferred molecular weight ranges as described for labeling groups also apply to the effector groups.

Preferred binding pair members comprise hapten, antigen and hormone. Especially preferred are haptens like digoxin and biotin and analogues thereof.

Charged linear linker molecules - or linker structures already carrying a marker - are synthesized essentially as described in US 5,958,783.

Branched charged linker molecules or linker (marker) molecules are e.g. obtained by solid phase synthesis. In a first step of the solid phase synthesis, an amino acid is coupled via its carboxylate group to the solid phase support and then the desired linker is synthesized by successively coupling further amino acids. In this process at least one amino acid which contains a charged group as a side group e.g. an amino group or a carboxylate group and at least one amino acid which serves as the branching site and is optionally in a protected form are used to prepare a linker according to the invention. After completion of the desired linker sequence the linker is either cleaved off the solid phase support or an activated marker, e.g. a marker carrying an active ester, can be coupled to the free N-terminal amino group of the peptide bound to the solid phase. After cleavage from the solid phase a reactive group Z can be coupled to the carboxy terminus of the peptide linker or the carboxy terminus itself is used to function as reactive group Z. Protective groups that may be present are cleaved off.

In another mode of solid phase synthesis an amino acid marker conjugate which contains a protected amino group and a carboxylate group e.g. Fmoc-Lys(-Ru(bipyridyl)$_3$-OH) can be anchored to a solid phase by means of the free carboxylate group and a peptide linker can be synthesized after release of the blocked amino group. After completion of the desired linker sequence, the complex is cleaved from the solid phase. The reactive group Z can be coupled to the amino terminus of the resulting peptide linker, or the amino terminus itself is used to function as reactive group Z.

Chemical coupling of the biomolecule on the one hand to a linker molecule on the other hand leads to a statistical mixture of various biomolecule-linker products. With other words, such conjugate comprises in statistical amounts biomolecules which are not conjugated at all, biomolecules comprising one linker structure, biomolecules comprising two linker structures and so on. Each of these biomolecule-linker product subgroups is characterized by a uniform stoichiometry. This means that, e.g., the subgroup comprising one linker structure per biomolecule has a uniform stoichiometry of 1:1.

For example, hapten-protein-conjugates can be synthesize by incubating a chemically activated hapten with a protein in a predefined molar ratio. Speciific coupling chemistries are known targeting different functional amino acid groups (-SH; -NH2; -COO$^-$; -OH; imino groups of tyrosine, imidazole groups of histidine). Usually these groups are repeatedly present in the protein. As a consequence, the hapten will not be evenly distributed but rather the hapten will be bound to the protein in a Poisson distribution. A Poisson distribution can be expressed as:

$$P(r) = m^r \times e^{-m} / r!$$

P(r) is the fraction of protein molecules with r (0,1,2,3, etc.) haptens bound per molecules; m is the average molar ratio between hapten and protein in the reaction mixture; e = Euler's number and r! represents faculty of r (e.g. in case of r = 3, r! =3 x 2 x 1). This means for example that for a crude reaction mixture comprising in the average 1 hapten molecule bound per protein molecule that statistically and roughly 36,7 % of the protein molecules are not labeled at all, 36,7% carry one hapten molecule, 18,3 % carry two hapten molecules, 6,1% carry three hapten molecules and the rest is even higher labeled.

It is an extremely attractive feature of the present invention that now conjugates between a biomolecule and a small marker group linked together by a linker molecule - which in itself in addition has advantageous effects on, e. g., background reduction in immunolgical assays - are now available in pre-determined and pre-defined composition.

Conjugates known from the art are usually characterized by a labeling index. This labeling index indicates the average amount of labels (linkers or markers) per biomolecule. A labeling index of 2.3 therefore signifies that in the overall mixture of biomolecule-linker products per biomolecule 2.3 labels are present. It is possible by sophisticated methods to determine the relative amounts of coupling products of uniform stoichiometry by complicated procedures, for example, MALDI-TOF (matrix assisted laser desorption ionization- time of flight) mass-spectroscopy. However, such methods are not appropriate for separating out fractions of uniform stoichiometry. The inventive procedures allow for the production of a conjugate comprising at least one pre-selected biomolecule linker product of uniform stoichiometry in non-statistical amounts.

The term "pre-selected" is used to indicate that the inventive conjugate is not the mere result of a chemical conjugation between a biomolecule and a linker molecule but rather that it is a result of pre-selection of appropriate fractions. One coupling product, or if required, several coupling products, of uniform stoichiometry can now be selected at will (as required) and the conjugate be composed as appropriate for the intended application.

The mode and strategy of chemical coupling can be selected as required. In case the biomolecule is a polypeptide comprising -SH, -NH$_2$ or -COO$^-$ residues as functional groups, several appropriate coupling chemistries are known form the art for each of these functional groups and only some shall be mentioned. The careful reader will find all required details in Aslam M., Dent A., Bioconjugation (1998) Mcmillan Reference Ltd., London, pages 216 to 363.

Amino groups of biomolecules (the terminal -NH$_2$ group or the NH$_2$ group of a lysine side chain, as well as ω-amino groups of diamino carboxylic acids) can be used for chemical coupling of a marker group thereto based on "amino chemistry". Well-known examples of amino chemistry comprise amongst others the reaction of amino groups with so-called activated groups, like NHS-esters, other activated esters, acid chlorides and azides.

Carboxyl groups on biomolecules (the terminal C00$^-$ - group, the carboxy functions of glutamic acid or aspartic acid) are used for chemical coupling based on "carboxy chemistry". Well-known examples of carboxy chemistry comprise amongst others the activation of these of carboxy groups to carry the above mentioned activated groups. Coupling to e.g., amino groups on the marker is then easily performed.

Alternatively sulfhydryl groups on biomolecules (e.g. free-SH-groups of cysteine or -SH groups obtained by reducing di-sulfhydryl bridges) are used for chemical coupling based on "sulfhydryl chemistry". Well-known examples of sulfhydryl chemistry comprise amongst others the reaction of -SH groups with maleimido groups, or alkylation with α-halogen carboxylic group or thioethers.

The hydroxyl group of tyrosine residues or the imidazol group of histidine also may be used to covalent link markers to a biomolecule by aid, e.g., of diazonium groups.

The chemical reaction for coupling the biomolecule to the linker or the linker (marker) is performed and if required, stopped as known from the art. Whereas in the art usually only the non-conjugated small linker or linker (marker) molecules are separated from the high molecular weight biomolecule-linker fraction. The separation of free biomolecule from conjugated biomolecule is very difficult. This separation and also the separation and selection of coupling products according to their stoichiometry is now possible and is performed by chromatographic procedures. The conjugation products are separated into fractions of uniform stoichiometry based on properties contributed by the linker structure.

Especially attractive properties of the novel biomolecule-linker products are differences in apparent molecular weight, hydrophobicity, and in charge. The differences for the various coupling products with different stoichiometry simply depend on the number of linker structures per biomolecule.

In a preferred embodiment the chromatographic fractionation is performed by a separation procedure based on (apparent) molecular weight. It was a very surprising finding that fractionation of the various coupling products with uniform but different stoichiometry is possible based on molecular weight, better said this separation is based on differences in apparent molecular weight. Despite absolute differences in molecular weight are comparatively small between coupling products of different stoichiometry, the differences in apparent molecular weight are quite pronounced. This is brought about by the linker (marker) molecules used in this invention. Although these molecules have a molecular weight of 1 to 15 kD, they migrate as having a very unusual and very high apparent molecular weight.

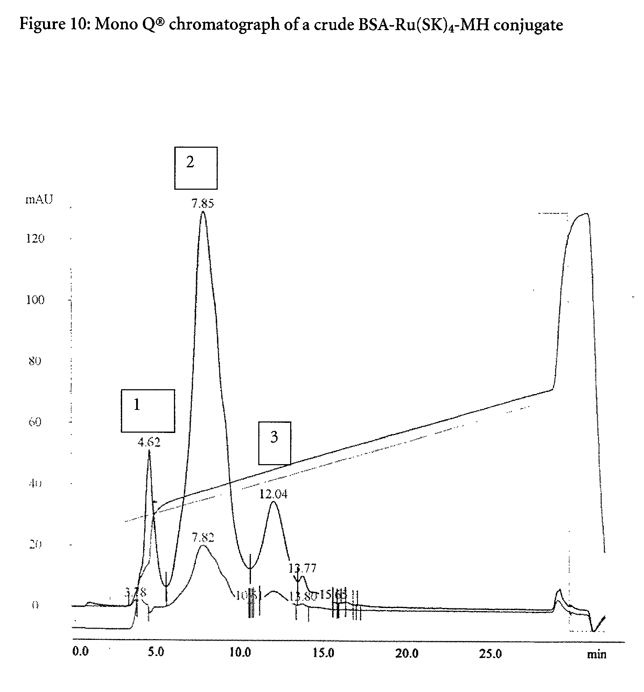

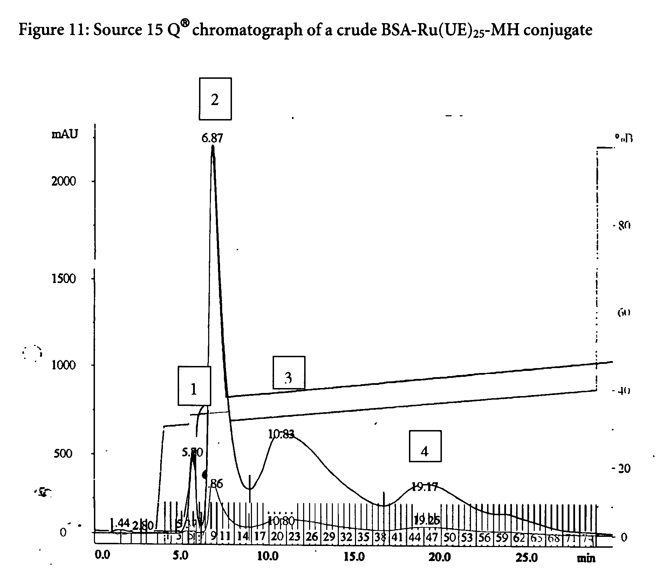

According to the invention it now is possible to easily separate and if required, fractionate, e.g., Fab'-fragments of a molecular weight of about 50 kD carrying one linker (label) molecule from Fab'-fragments carrying none, two, three, or more linker (label) molecules. This is exemplified in Examples 4 and 5 and in Figures 10 and 11. Such separation is possible, because the linker structures of the biomolecule-linker conjugates according to the present invention have been found to have apparent molecular weight which is much higher as their real molecular weight.

It is possible to separate the coupling products of uniform stoichiometry between even larger biomolecules, like e.g., $F(ab')_2$-fragments (about 100 kD), BSA or immoglobulin G and a linker (marker) molecule once coupled to an appropriate linker (marker).

The apparent molecular weight for a biomolecule can easily be determined according to procedures known in the art. Routinely used is, e.g., molecular sieve chromatography. The molecule of interest is chromatographed and the apparent molecular weight determined by comparing the retention time for this molecule to the retention time or times of one or several molecular weight markers.

Since the apparent molecular weight is greatly influenced by the procedures used for determination, the apparent molecular weight of the biomolecule and the apparent molecular weight of the linker (marker) structure both are determined by the same procedure.

For ease of separation by molecular weight chromatography, linkers are chosen which match the apparent molecular weight of the biomolecule. Preferably, the apparent molecular weight of the linker (marker) is at least 20 % and at most 500 % of the molecular weight of the biomolecule. A process for the production of a conjugate comprising at least one biomolecule-linker product of uniform stoichiometry in a pre-selected amount, said conjugate consisting of a biomolecule of a molecular weight between 5 kD and 500 kD and a hydrophilic linker molecule, said linker having a molecular weight between 1 kD and 15 kD, between 4 and 60 charged residues and an apparent molecular weight between 20 % and 500 % of the apparent molecular weight of said biomolecule, characterized in that

a) the biomolecule and the linker molecule are covalent coupled to each other,

b) the different biomolecule-linker products of uniform stoichiometry are fractionated by chromatography based on the differences in apparent molecular weight of said biomolecule-linker products, and

c) fractions containing a coupling product of uniform stoichiometry are collected,

therefore represents a preferred embodiment of the invention. More preferred the apparent molecular weight of the linker (marker) is between 25 % and 400 %, most preferred between 30 % and 330 % of the apparent molecular weight of the biomolecule.

Separation by (apparent) molecular weight preferably is performed by molecular sieve chromatography using chromatography materials like Sephadex S 200 HR®.

A further striking and characteristic feature of the linker structure is the fact that they carry at least four charged residues per molecule. The high number of charged residues within the linker also leads to differences in net charge of the biomolecule-linker products, these charge differences being dependent on the number of linkers per biomolecule. Biomolecule-linker products with one linker per biomolecule (of uniform 1:1 stoichiometry) can easily be separated from biomolecule-linker products of different stoichiometry or from biomolecules without any linker attached.

In a preferred embodiment the invention relates to a process for the production of a conjugate comprising at least one biomolecule-linker product of uniform stoichiometry in a pre-selected amount, said conjugate consisting of a biomolecule of a molecular weight between 5 kD and 500 kD and a hydrophilic linker molecule, said linker having a molecular weight between 1 kD and 15 kD, between 4 and 60 charged residues, characterized in that

a) the biomolecule and the linker molecule are covalent coupled to each other,

b) the different biomolecule-linker products of uniform stoichiometry are fractionated by chromatography, based on differences in charge of said biomolecule-linker product.

c) fractions containing a coupling product of uniform stoichiometry are collected.

Preferably, standard chromatography materials and procedures, like ion exchange chromatography , e.g., employing Mono Q®, Mono S®, Source Q® or Source S® from Amersham-Pharmacia Biotech, are used to separate biomolecule-linker products of uniform stoichiometry based on charge differences. Fractions containing the desired products are collected. Individual biomolecule linker products of pre-selected stoichiometry or mixtures of several products of pre-selected stoichiometry and in pre-selected relative amounts are obtained. If desired, higher purifies are obtained be rechromatography. Any desired pre-selected ratio of conjugates is obtained by pooling appropriate fractions.

As mentioned above the coupling products of different stoichiometry also are different with respect to their hydrophilicity. The hydrophilic linker structures influence the overall hydrophilicity or hydrophobicity of the coupling products. Products of different stoichiometry in a preferred embodiment also are separated by hydrophobic interaction chromatography using state of the art resins, like Phenyl Sepharose HP®, Butyl Sepharose 4 fast flow® or others. Essential materials and procedures are described in Aslam M., Dent A., Bioconjugation (1998) Mcmillan Reference Ltd., London, p 620 to 626. Reversed phase chromatography may also be used.

The conjugate produced according to the procedures described herein contains at least one uniform coupling product between a biomolecule and a linker molecule in the amounts desired. The relative amounts of each coupling product of uniform stoichiometry can be pre-selected as required. Pre-determined, desired relative amounts of individual coupling products of uniform stoichiometry can be exactly and reproducibly adjusted.

In a further preferred embodiment the pre-selected conjugate is essentially free of non-conjugated biomolecules. It is also preferred that based on the fractionation now possible, the inventive conjugate does not contain biomolecule-linker products in a stoichiometry of 1:5 and above.

In a preferred embodiment the invention relates to a conjugate consisting of a biomolecule of a molecular weight between 5 kD and 500 kD and a hydrophilic linker molecule said linker having a molecular weight between 1 and 15 kD and between 4 and 60 charged residues, characterized in that said conjugate comprises at least one biomolecule-linker product of uniform stoichiometry in a pre-selected amount.

Any desired purity of individual coupling products of uniform stoichiometry can now be produced and reproduced. Mixtures containing various coupling products of uniform stoichiometry in ratios (relative amounts) as required can easily be produced and reproduced. The inventive conjugate therefore comprises at least one biomolecule-linker product of uniform stoichiometry in a pre-selected relative amount as compared to at least one other biomolecule-linker product of uniform stoichiometry.

The less biomolecule-linker products of uniform stoichiometry form part of the conjugate the more easy it is produced or reproduced. A preferred conjugate essentially comprises 1 to 3 individual biomolecule-linker products of uniform stoichiometry. Even more preferred the conjugate comprises essentially two different coupling products of uniform stoichiometry. In a most preferred embodiment the conjugate according to the present invention essentially comprises a biomolecule-linker product of 1:1 stoichiometry.

In a further preferred embodiment the conjugate according to the present invention essentially comprises only one biomolecule-linker product of uniform stoichiometry.

Amongst the various coupling products those with a uniform stoichiometry between 1:1 and 1:4 are preferred. Especially preferred are the biomolecule linker products with 1:2 and 1:1 stoichiometry, the latter representing the most preferred coupling product of uniform stoichiometry.

The term "essentially comprises" refers to the relative amount of one coupling product (or a mixture of up to three coupling products (each) of uniform stoichiometry as compared to the relative amount of other coupling products. Preferably essentially comprises relates to conjugates containing at least 80 % of the uniform coupling product (or mixture of coupling products). More preferred are conjugates comprising 90 % of the pre-selected coupling-product or mixture of coupling products. Most preferred is a relative purity of 95 % for the selected conjugate(s).

In another preferred embodiment the conjugate comprises a relative amount of at least 80 % of two and more preferred of only one biomolecule-linker product of uniform stoichiometry.

A preferred conjugate comprises the 1:1 coupling product of a biomolecule with a molecular weight between 5 kD and 500 kD and a hydrophilic linker molecule said linker having a molecular weight between 1 and 15 kD and between 4 and 60 charged residues, characterized in that said conjugate comprises at least one pre-selected biomolecule-linker product of uniform stoichiometry in a purity, i.e. in a relative amount of 80 % or above as compared to the sum of other coupling products.

More preferred is a conjugate comprising the coupling product with 1:1 stoichiometry in a relative amount of 90 % or above, even more preferred are conjugates comprising the coupling product of 1:1 stoichiometry in a relative amount of 95 % or above as compared to coupling products of different stoichiometry. The term "purity" is used to indicate that pre-selected or predetermined relative amount(s) of one (to three) biomolecule linker product(s) is (are) present in the conjugate as compared to the other coupling products.

Nowadays, commercial assays are manufactured to require as little handling steps as possible. This is convenient and reduces the chances of handling errors. Of course, it is possible to provide the conjugate of the invention as such, e.g., frozen, or lyophilized to the customer.

It is preferred that the conjugate is part of a reagent composition. In many cases the conjugate is present in dilute form and therefore stabilizers, e.g. bovine serum albumin and/or various sugars and optionally as well reagents like preservatives and/or detergents are present. In a further preferred embodiment the invention relates to a composition of reagents comprising buffer components, a stabilizing reagent and a conjugate containing at least one biomolecule-linker product of uniform stoichiometry in a pre-selected amount.

Preferably the conjugate as produced according to the present invention is used in a test strip type immunological device.

Usually, not single reagent but rather complete kits, containing at least the analyte-specific assay reagents are provided to the customer. In a preferred embodiment the invention relates to a test kit for biochemical or immunological detection of an analyte in a sample, said kit comprising appropriate buffers and reagents and at least one composition of reagents comprising a conjugate according to the present invention.

Immunoassays based on the novel conjugates have superior characteristics, as compared to assays based on conjugates produced with methods in the art. This positive effect on the assay itself is shown in the Examples section. Strikingly, the background problem is significantly reduced if the novel conjugate is used under otherwise comparable assay conditions. The use of a conjugate comprising as biomolecule a member of a specific binding pair as described in the present invention in an immunoassay therefore represents another preferred embodiment of the invention.

Preferably, the novel conjugate is used in the detection of an analyte which is based on the reaction between an analyte specific binding partner and the analyte and the determination of the resulting analyte binding partner complex. Best known examples of specific binding assays are immunoassays. In immunoassays based on a biomolecule-linker (marker) conjugate the novel processes and reagents contribute to reproducibility of conjugates. The more reproducible the conjugates, the better reproducible are the assays based thereon.

As discussed, both quality of conjugate as well as reproducibility according to state of the art procedures can only be influenced to a limited extend. It is, e.g., possible to remove aggregates or precipitates from the final conjugate. However, it is not possible to adjust the relative amounts of the coupling products of different stoichiometry between the biomolecule and the linker molecule. It is very difficult or not possible to select the fraction(s) containing those coupling product(s) of uniform stoichiometry which is (are) best suited for the intended application.

The following examples, references, and figures are provided to aid the understanding of the present invention, the true scope of which is set forth in the appended claims. It is understood that modifications can be made in the procedures set forth without departing from the spirit of the invention.

**<u>Legend to the Figures:</u>**

**Figure 1: Schematic representation of coupling BSA and the hy-BPRu-linker**

The schematic for BSA illustrates the fact that several -NH$_2$ groups are available for binding to the hy BPRu-linker (marker).

**Figure 2: Superdex 200 HR® chromatograph of a BSA-hy-BPRu-conjugate**

This chromatograph demonstrates that the coupling products with different stoichiometries migrate as a single uniform peak. This single peak represents a statistical mixture of coupling products.

**Figure 3: Schematic of BPRu-(UE)$_{25}$ K and BPRu -(UE)$_{50}$ K**

These linker structures repeatedly contain the di-peptide β-alanine-glutamic acid (UE).

**Figure 4: Branched linker with short backbone chain**

This branched linker contains charged residues both in the short backbone, as well as in the side chains.

**Figure 5: Branched linker with long backbone chain**

This branched linker contains charged residues both in the long backbone, as well as in the side chains.

**Figure 6: Branched linker without charged residues**

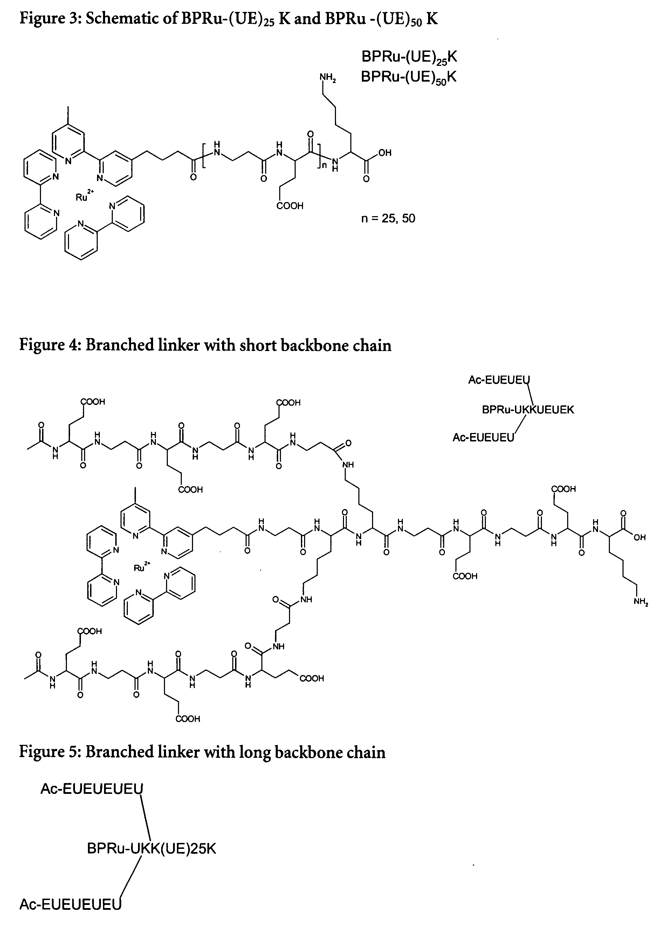

This linker is very similar to the linker of Fig. 4 but does not carry charged residues of glutamic acid, instead glutamine is used. The apparent molecular weight has been found to be about 3,5 kD as compared to 18 kD for the linker of Fig. 4.

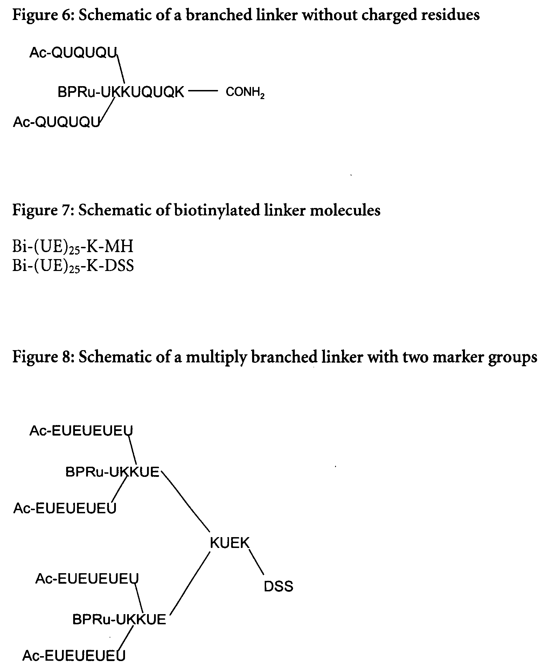

**Figure 7: Schematic of biotinylated linker molecules**

These N-terminally biotinylated linker molecules carry at the C-terminus -MH and -DSS groups respectively, which are used for coupling to a biomolecule.

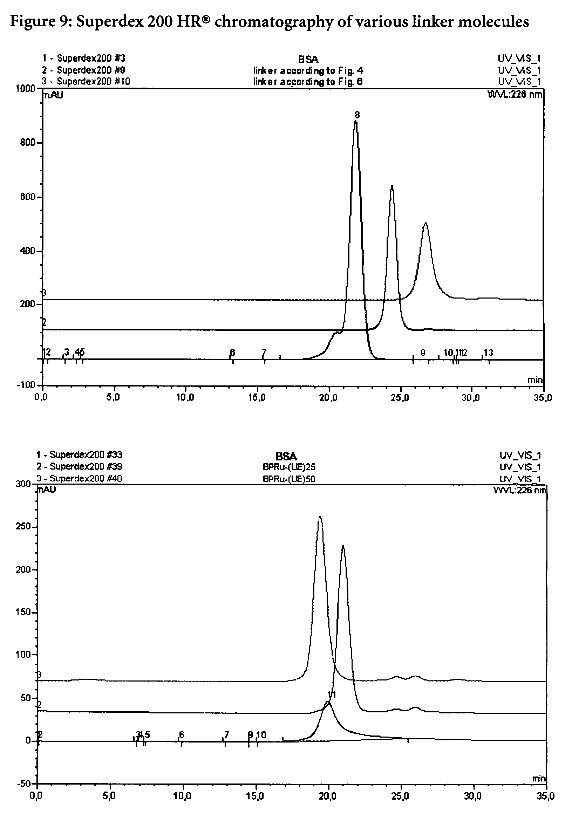

**Figure 8: Multiply branched linker**

This schematic depicts a multiply branched linker with branched side chains, carrying two ruthenium labels (BPRu).

**Figure 9: Superdex 200 HR® chromatography of various linker molecules**

BSA and the different linker molecules have been chromatographed under identical conditions. The upper panel shows linker structures according to Figures 4 and 6. The lower panel shows linker structures according to Figure 3.

**Figure 10: Mono Q® chromatograph of a crude BSA-Ru(SK)$_4$-MH conjugate**

The chromatograph shows baseline separation of BSA (1) and conjugation products of uniform 1:1 (2) and 1:2 (3) stoichiometry, respectively.

**Figure 11: Source 15 Q® chromatograph of a crude BSA-Ru(UE)$_{25}$-MH conjugate**

Three conjugates of uniform stoichiometry 1:1; 1:2 and 1:3 (2, 3 and 4, respectively) are clearly separated from one another, as well as from non-conjugated BSA (1).

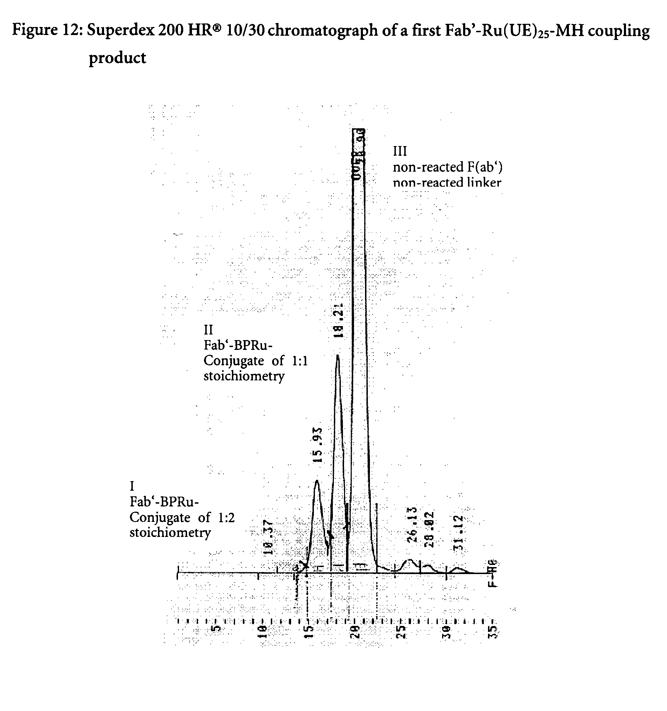

**Figure 12: Superdex 200 HR® chromatograph of a first Fab'-Ru(UE)$_{25}$-MH coupling product**

The crude conjugate is separated into three major peaks. Peak III which migrates with an apparent molecular weight of 50 kD comprises both the non-reacted Fab' as well as non-reacted linker (Ru-(UE)$_{25}$-MH). Peak II, which migrates with an apparent molecular weight of 100 kD comprises in essentially pure form the 1:1 coupling product between Fab' and linker. Peak I comprises the 1:2 coupling product (apparent molecular weight of 150 kD). Compositions of each pool has been confirmed by MALDI-TOF MS.

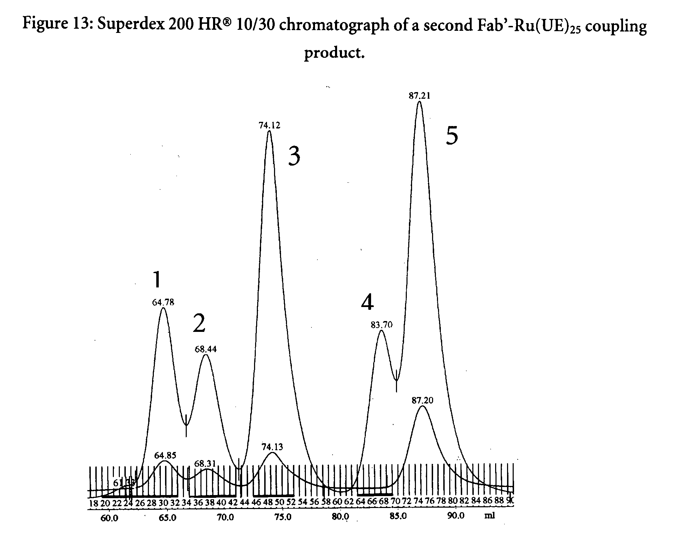

**Figure 13: Superdex 200 HR® chromatograph of a second Fab'-Ru(UE)$_{25}$ coupling product.**

Peaks 1, 2 and 3 correspond to fractions containing conjugates of 1:3, 1:2 and 1:1 stoichiometry, respectively. Peaks 4 and 5 contain the un-conjugated starting materials (linker and Fab'-fragments). All structures have been confirmed by MALDI-TOF-MS.

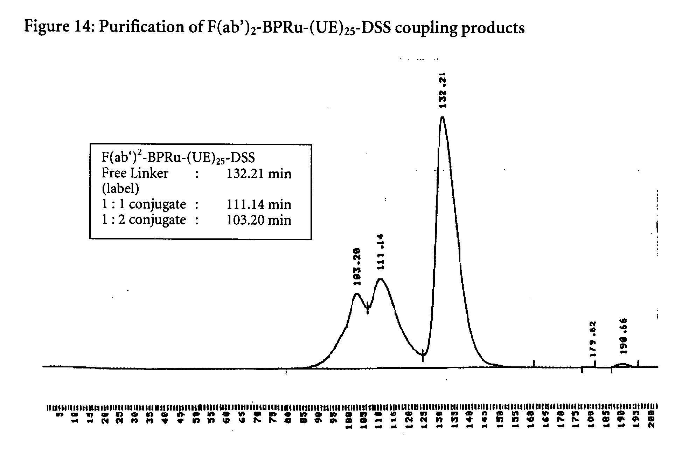

**Figure 14: Purification of F(ab')$_2$-BPRu-(UE)$_{25}$-DSS coupling products**

Crude conjugate is separated by Superdex 200 HR® chromatography. The peaks containing the 1:2 and the 1:1 coupling products are pooled separately and re-chromatographed. The composition of these coupling products has been confirmed by MALDI-TOF-MS.

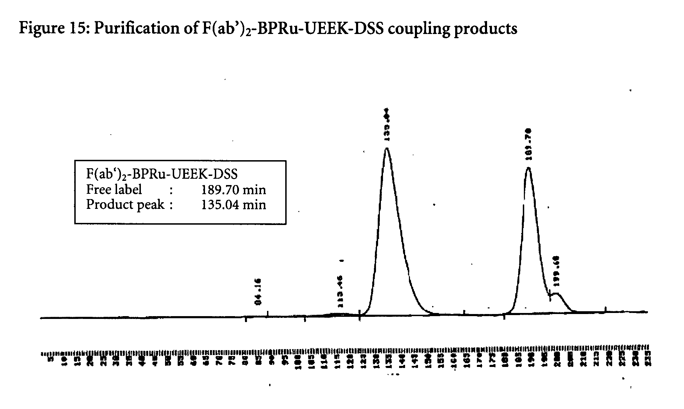

**Figure 15: Purification of F(ab')$_2$-BPRu-UEEK-DSS coupling products**

Crude conjugate is separated by Superdex 200 HR® chromatography. Only one symmetric "product peak" is found, comprising a statistical mixture of coupling products with different stoichiometries.

**Example 1: Ruthenylation of bovine serum albumine according to the state of the art**

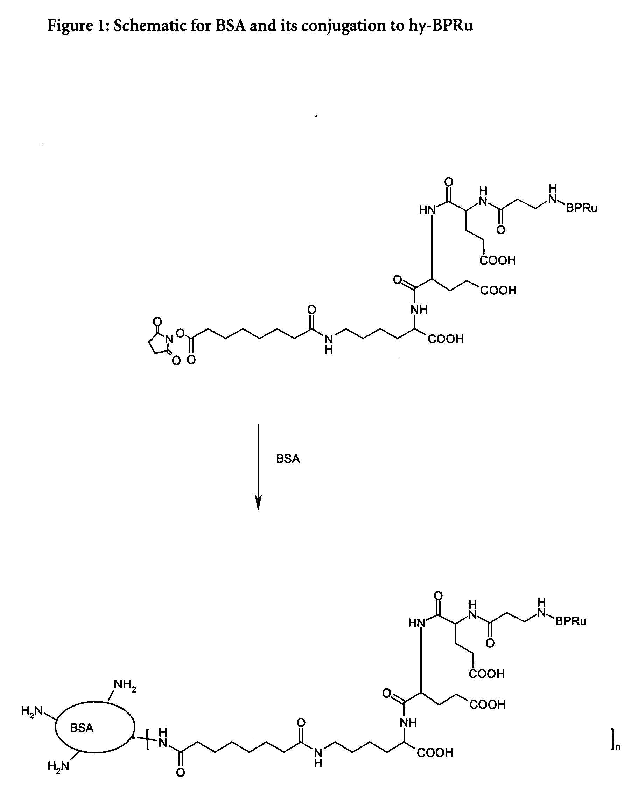

Bovine serum albumine and a linker known in the art (see Figure 1) have been used to set up 5 conjugation mixtures. All variables like lot-size, concentrations of reagents, buffer compositions, time of reaction, temperature of reagents as well as chromatographic separation procedures have been kept constant.

BSA was dissolved in a 100 mM potassium phosphate buffer (pH 8.0) at a concentration of 20 mg/ml. The hy-BPRu linker (label) was dissolved in DMSO at a concentration of 47 mg/ml. A 5-fold molar excess of hy-PBRu was added to the BSA solution, the reagents thoroughly mixed and reaction performed for 75 min at 25°C. The reaction was stopped by addition of lysine to a final concentration of 10 mM and incubation under stirring for further 30 min at 25°C.

Free non-bound derivatization reagents were completely removed by dialysis (20 hours at 4°C against phosphate buffered saline with 50 mM phosphate 150 mM sodium chloride at pH 7.5 (PBS), which has been used in 500-fold excess.

The active group the O-hydroxysuccinimide ester in Figure 1 react statistically with the different $NH_2$-groups of BSA. The resulting crude conjugate has been chromatographed by Superdex 200 HR® chromatography using buffer conditions of 0.05 mmol/l sodium phosphate, 0.05 mmol/l sodium chloride and 5 % methanol at pH 6.5. Elution is monitored. Appropriate fractions are pooled. Fractions were analyzed by electrosprayionization mass-spectroscopy (ESI-MS). The result of such analysis are summarized in Tables 1 and 2.

Table 1:

| Distribution of BSA-Ruthenium conjugates according to molecular weight | | | | | | |
|---|---|---|---|---|---|---|
| Lot | Labeling index RSA: BPRu | Stoichiometry 1:0 | Stoichiometry 1:1 | Stoichiometry 1:2 | Stoichiometry 1:3 | Stoichiometry 1:4 |
| BSA unmodified | 0 | 66500 D | | | | |
| Conjugate Ch1 | 1: 2,3 | | 68300 D | 69400 D | 70400 D | 71300 D |
| Conjugate Ch2 | 1:2,3 | 66500 D | 68300 D | 69500 D | 70500 D | |
| Conjugate Ch3 | 1:2,8 | | 68500 D | 69600 D | 70700 D | 71800 D |
| Conjugate Ch4 | 1:2,9 | | 68000 D | 69200 D | 70300 D | |
| Conjugate Ch5 | 1:3,1 | | | 69400 D | 70600 D | 71500 D |

Table 2:

| Relative amounts of the different coupling products (the most abundant coupling product has been set to 1 and the others are expressed as relative amounts thereto). | | | | | | |
|---|---|---|---|---|---|---|
| Lot | Labeling index RSA: BPRu | Stoichiometry 1:0 | Stoichiometry 1:1 | Stoichiometry 1:2 | Stoichiometry 1:3 | Stoichiometry 1:4 |
| BSA unmodified | 0 | 1,00 | | | | |
| Conjugate Ch1 | 1: 2,3 | | 0,77 | 1,00 | 0,57 | 0,40 |
| Conjugate Ch2 | 1:2,3 | 0,48 | 0,48 | 1,00 | 0,71 | |
| Conjugate Ch3 | 1:2,8 | | 0,38 | 0,78 | 1,00 | 0,62 |
| Conjugate Ch4 | 1:2,9 | | 0,59 | 1,00 | 0,57 | |
| Conjugate Ch5 | 1:3,1 | | | 0,54 | 1,00 | 0,56 |

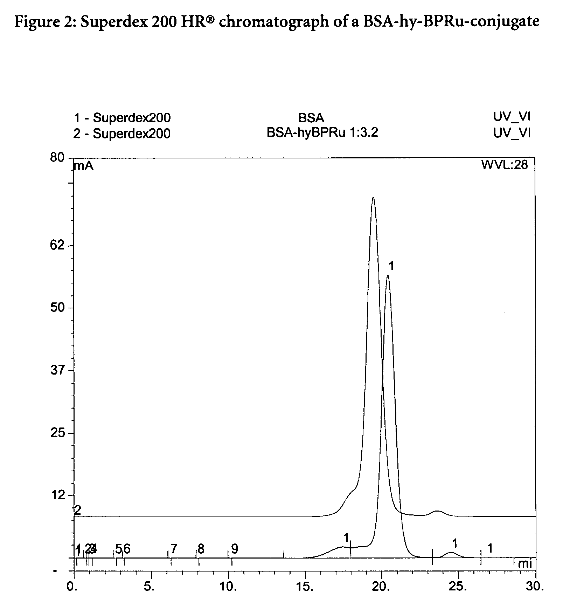

It is obvious from tables 1 and 2 that all 5 lots, though produced under identical conditions clearly are different from another. Lot 1 and 2 have the same overall labeling index of 2.3 (this means in the average 2.3 ruthenium labels are found per BSA molecule). Nonetheless, both preparations differ significantly in the relative amounts of the individual conjugation products as is evident from Table 2. Also the overall labeling indices vary quite significantly. It is evident from Figure 2 that by standard chromatographic procedures using a Superdex 200 HR® column, all reaction products elute as a homogeneous peak. Fractionation of coupling products e.g., into a fraction containing uniformly a 1:1 conjugate, is not possible.

## Example 2: Synthesis of linker structures

### Synthesis of branched linker structures

Preparation of branched linkers by means of solid phase peptide synthesis

The branched linkers were synthesized by means offluorenylmethyloxycarbonyl-(Fmoc)-solid phase peptide synthesis on a batch peptide synthesizer e.g. from Applied Biosystems A433. In each case 4.0 equivalents of the amino acid derivative shown in table 3 were used for this.

Table 3:

| | |
|---|---|
| A | Fmoc-Ala-OH |
| C | Fmoc-Cys(Trt)-OH |
| D | Fmoc-Asp(OtBu)-OH |
| E | Fmoc-Glu(OtBu)-OH |
| gE | Fmoc-Glu-OtBu |
| F | Fmoc-Phe-OH |
| G | Fmoc-Gly-OH |
| H | Fmoc-His(Trt)-OH |
| I | Fmoc-Ile-OH |
| K1 | Fmoc-Lys(Boc)-OH |

Table 3: (continued)

| K2 | Fmoc-Lys(Fmoc)-OH |
| K3 | Fmoc-Lys(Dde)-OH |
| K4 | Fmoc-Lys(Alloc)-OH |
| K5 | Fmoc-Lys(PhAc)-OH |
| K6 | Fmoc-Lys-(label)-OH |
| K7 | Boc-Lys(Fmoc)-OH |
| L | Fmoc-Leu-OH |
| M | Fmoc-Met-OH |
| N | Fmoc-Asn(Trt)-OH |
| P | Fmoc-Pro-OH |
| Q | Fmoc-Gln(Trt)-OH |
| R | Fmoc-Arg(Pmc)-OH |
| S | Fmoc-Ser(tBu)-OH |
| T | Fmoc-Thr(tBU)-OH |
| U | Fmoc-α-alanine-OH |
| V | Fmoc-Val-OH |
| W | Fmoc-Trp-OH |
| Y | Fmoc-Tyr(tBU)-OH |
| Z | Fmoc-α-amino caproic acid |
| Ps | Fmoc-Ser(PO(OBzl)OH)-OH |
| Cs | Fmoc-Cys(SO3H)-OH |

The amino acids and amino acid derivatives were dissolved in N-methyl-pyrrolidon. The peptide is synthesized on Wang resin (S.-S. Wang, J. Am. Chem. Soc. 95 (1973) 1328). The resin is loaded with 0.2 to 0.4 mMol/g. The coupling reactions were carried out for 20 minutes using 4 equivalents dicyclohexylcarbodiimide and 4 equivalents N-hydroxybenzotriazole in dimethyl-formamide relative to the Fmoc-amino acid derivative in dimethylformamide as the reaction medium. The Fmoc group was cleaved after each step of the synthesis with 20 % piperidine in dimethylformamide for 20 min. The amount of resin was selected such that after the last branch, 4 equivalents Fmoc-amino acid relative to the amino groups are used. Fmoc-Lys(Fmoc)-OH is used for the branch and subsequent synthesis of two identical arms. Non-symmetric branches are achieved by amino acid derivatives with orthogonal side chain protective groups such as Fmoc-Lys(Dde) or Fmoc-Lys(Alloc). These orthogonal protective groups are cleaved on the resin by methods known in the literature (B.W. Bycroft et al. (1993), J. Chem. Soc., Chem. Commun., 778; A. Merzouk et al., (1992) Tetrahedron Lett. 33, 477). Terminal amino groups on the solid phase are optionally acetylated or succinylated with acetic anhydride or succinic anhydride.

The hapten, label or functional group was already introduced on the resin e.g. on the N-terminal amino acid of the peptide if the derivative is stable during the solid phase synthesis.

The introduction of e.g. a metal chelate label was carried out via appropriate active ester derivatives at the free N-terminal amino group of the carrier-bound peptide. For this four equivalents ruthenium(bipyridyl)3 complex (BPRu) per free primary amino function were activated with N-hydroxybenzotriazole/dicyclohexyl-carbodiimide and dissolved in a small amount of DMSO and this was added drop-wise and stirred for 2 h at room temperature.

The marker can also be introduced at the C-terminus already during the solid phase synthesis by the direct incorporation of for example a metal chelate or biotin-coupled amino acid derivatives (described in WO 96/03409).

The peptide is released from the support and the acid-labile protective groups are cleaved with 20 ml trifluoroacetic acid, 0.5 ml ethanediol, 1 ml thioanisole, 1.5 g phenol and 1 ml water within 40 min at room temperature. Depending on the amino acid derivatives that are used, it is also possible to use cocktails containing fewer radical traps. 300 ml cooled diisopropyl ether was subsequently added to the reaction solution and was kept for 40 min at 0°C in order to completely precipitate the peptide. The precipitate was filtered, washed with diisopropyl ether and dissolved in a small amount of 50 % acetic acid and lyophilized. The crude material obtained was purified by means of preparative HPLC on Delta-PAK RP C18 (column 50 x 300 mm, 100 Å; 15 μ) over an appropriate gradient (eluant A: water, 0.1 % trifluoroacetic acid, eluant B: acetonitrile, 0.1 % trifluoroacetic acid) within ca. 120 min. The eluted material was identified by mass spectrometry.

Alternatively the marker group can also be introduced after cleavage from the resin. For this it may be necessary to block other groups that should not be derivatized with a protective group which is stable during the solid phase peptide synthesis as well as during the cleavage (e.g. phenylacetyl (Phac)). The protective group can be removed enzymatically with PenG amidase (described in PCT/EP 95/02921).

**b) Synthesis of linear charged linker structures**

Linear charged linker structures have been synthesized essentially as described in US 5,958,783 .

**c) Examples of linker structures investigated.**

Table 4 gives an overview over some of the linker structures used. Structure 1 and 10 are structures know from the art, whereas all other structures comprise additional features, e.g., a strikingly high apparent molecular weight.
Where available, laboratory names have also been given. References are made to the Figures in which these structures (basic structures used for MH- or DSS-activation) are given.

Table 4:

| No. | Lab-name | Fig | MW [D] | MW (a) |
|-----|----------|-----|--------|--------|
| Overview of linker structures (linear or branched) |
| 1 | hy-BPRu | 1 | 1773 | ∼ 2 kD |
| 2 | BPRu(UE)$_{25}$ K | 3 | 5802 | ∼ 50 kD |
| 3 | BPRu(UE)$_{50}$ K | 3 | 10807 | ∼ 70 kD |
| 4 | BPRu(UE)$_{25}$-K-MH | [3] | 5996 | ∼ 50 kD |
| 5 | BPRu(UE)$_{25}$-K-DSS | [3] | 6056 | ∼ 50 kD |
| 6 | BPRu(UE)$_{50}$-K-MH | [3] | 11001 | ∼ 70 kD |
| 7 | BPRu-SK(2) charged | 4 | 3004 | ∼ 18 kD |
| 8 | BPRu(UE)$_{25}$-K-MH branched | 5 | 8010 | ∼ 70 kD |
| 9 | BPRu(UE)$_{25}$-K-DSS branched | 5 | 8072 | ∼ 70 kD |
| 10 | BPRu-SK(2) not charged | 6 | 3392 | 3,5 kD |
| 11 | BPRu(UE)$_{25}$-K-MH | [7] | 5570 | ∼ 50 kD |
| 12 | BPRu(UE)$_{25}$-K-DSS | [7] | 5630 | ∼ 50 kD |
| 13 | BPRu(SK)$_4$-DSS multiply branched linker | 8 | 6457 | ∼ 60 kD |

(a)      Apparent molecular weight
n.d.      not determined
n.s.      not shown
MH      maleimide activated
DSS      N-hydroxysuccinimidylsuberate
Bi      biotin
[ ]      basic structure shown in Figure [ ]

Figure 9 shows Superdex 200 HR® chromatographs of linker (marker) structures in comparison to BSA. The dramatic influence of charged residues becomes evident from the top panel, whereas the uncharged linker migrates with a molecular weight of about 3 500 D the charged linker is found with an apparent molecular weight of about 18 000 D. Figure 9 bottom panel demonstrates the great effects which a long charged backbone of a linker molecule has on differences in apparent molecular weight. Structure 2 (BPRu-(UE)$_{25}$) which has a molecular weight of 5 802 for example migrates with an apparent molecular weight of around 50.000 D.
As already indicated in table 3 the linker molecules may be activated. An example for activation is the introduction of a maleineimido function. In order to introduce the maleinimide function, a linker e.g. produced according to example 2 is dissolved in 0.1 M potassium phosphate buffer pH 7.0 and admixed with one equivalent maleinimidohexanoic acid N-hydroxysuccinimide ester (dissolved in DMSO) and stirred for 16 h at 25°C. The preparation is purified by preparative HPLC using an RPC18 column (see above). The identity of the eluted material is checked by means of mass spectrometry.

## Example 3: Novel bovine albumine linker conjugates

As described in WO 96/03652 carrier molecules comprising multimeric antigens are of great advantage in the detection of anti-viral-antibodies. For production of a polyhapten, the amino groups of carrier molecule first are activated using a active ester group. This active ester group is selected to match the corresponding active group on the marker group used. Preferred active groups are the maleinimidohexyl (MHS) or maleinimidopropyl-N-hydroxysuccinimide ester (MPS). As a result primary amino groups in the carrier (for example the ε-amino side chains of lysine residues) partially are derivatized to carry the desired maleinimido groups. After coupling the marker group to the activated carrier the $-NH_2$ groups of the carrier are present either unmodified, or carrying an MHS group, or carrying a label via the MHS-linker.

The linker (marker) structures schematically shown in Figures 7 and 8 have been coupled to BSA under identical conditions.

BSA is dissolved in 0.1 mmol/l potassium phosphate buffer of pH 7.5 in a concentration of 10 mg/ml. The linker (marker) is added in a molar ratio of 4.5 per mole of BSA. Reaction is performed for 3 hours at room temperature. The reaction product is subjected to ion exchange chromatography. Separation of coupling products with uniform stoichiometry is achieved by using a Source 15 Q® or a Mono Q® column.

The crude conjugate of the multiply branched linker (cf. Fig. 8 and structure 13 of table 1) was fractionated with Mono Q® chromatography. Elution was performed using a salt gradient from 20 mM potassium phosphate buffer (pH 6.5) (= eluent A) to 1M sodium chloride in 20 mM potassium phosphate buffer (pH 6.5) (= eluent B). Elution is monitored at 280 nm. The gradient shown in Figure 10 covers the range of 25 to 54 % eluent B and corresponds to 30 min of elution. Appropriate fractions are pooled. Fractions are analyzed by MALDI-TOF-MS. MS data confirmed that each of the three peaks contained in pure form BSA (1 in Fig. 10) and BSA molecules carrying 1, or 2 linker (marker) groups (2, and 3 in Fig. 10, respectively).

The crude conjugate obtained with the long and strongly negative charged linear linker (cf. Structure 12 from table 4 and Fig. 3) has been separated by Source 15 Q® chromatography. Elution was performed using a salt gradient from 20 mM potassium phosphate buffer (pH 6.5) (= eluent A) to 1M sodium chloride in 20 mM potassium phosphate buffer (pH 6.5) (= eluent B). Elution is monitored at 280 nm. The gradient shown in Figure 10 covers the range of 30 to 40 % eluent B and corresponds to 30 min of elution. Appropriate fractions are pooled. Fractions are analyzed by MALDI-TOF-MS. MS data confirmed that each of the four peaks contained in pure form BSA (1 in Fig. 11) and BSA molecules carrying 1, 2 or 3 linker (marker) groups (2, 3 and 4 in Fig. 11, respectively).

## Example 4: Production of a first novel Fab'-linker-conjugate

### 1. Description of the procedure

### 1.1. Preparation of the Fab' from IgG

A monoclonal antibody to digoxin (anti-Dig) was cleaved by pepsin to form $F(ab')_2$. After quantitative cleavage the pepsin was inactivated by increasing the pH and adding pepstatin. The $F(ab')_2$ was reduced by means of cysteamine to Fab' without prior purification. Cysteamine cleaves almost selectively the disulfide bridges in the hinge region. It was subsequently dialysed. This removes most of the Fc cleavage products generated by pepsin since they are small enough to pass through the pores of the dialysis tube (> 10,000 Dalton).

### 1.2. Fab'-BPRU linker conjugate.

The conjugate synthesis was carried out via -SH group chemistry by reacting the Fab' with an excess of BPRu-linker-MH. In this process an SH group in the hinge region was mainly converted. Small amounts of polyruthenylated Fab' were formed as a side reaction as a result of reduced intramolecular disulphide bridges in the light and in the Fd chain.

### 1.3. Purification of the crude conjugate

The crude conjugate was purified by a molecular sieve. In this process the mono-ruthenylated material was separated from the poly-ruthenylated material.

## 2. Procedure

### 2.1. Cleavage of the antibody to form F(ab')$_2$

The lyophilisate of the monoclonal antibody anti-DIG-M19.11 IgG was reconstituted with H2O to obtain a concentration of 20 mg/ml. 20 µl 1 M citrate pH 3.5 were added per ml solution (final concentration citrate = 20 mM). The pH was adjusted with HCl to 3.60. It was filtered through a 0.45 µm filter. The concentration was determined at OD 280 nm (1 OD280nm = 1.4 mg/ml). It was adjusted to 10 mg/ml with 20 mM citrate pH 3.60. The solution was heated in a water bath to 37°C. 100 µl pepsin solution (3 mg/ml) was added per ml antibody solution and incubated at 37°C in a water bath. After complete cleavage the reaction was stopped by increasing the pH value and adding pepstatin.

### 2.2. Reduction to Fab'

52.6 µl 0.1 M dithiothreitol (DTT) was added per ml cleavage mixture and incubated for 30 min at 25°C in a water bath. The Fab' was dialyzed against 0.1 M NaH2PO4/NaOH pH 6.5, 30 mM NaCl, 2 mM EDTA.

### 2.3. Synthesis of the Fab'-BPRu-linker conjugate

The BPRu-linker-MH was dissolved in DMSO. The stoichiometry Fab':BPRu-linker-MH was 1:3 (mole/mole). The final concentration of Fab' in the mixture was 3.9 mg/ml. The maximum concentration of DMSO in the mixture was 10 %. The reaction time was 1 h at room temperature.

### 2.4. Purification

The crude conjugate was concentrated 2-3-fold using an Amicon PM 10 and purified by means of Superdex 200 HR® (buffer: 25 mM MOPS/NaOH pH 6.5, 50 mM NaCl, 10 % DMSO; applied amount: max 1.5 % of the gel bed, fractions: 0.5 % of the gel bed). The fractions containing the Fab'-BPRu-linker conjugate were pooled. If required, rechromatography can be performed.
The crude conjugate predominantly contains coupling products consisting of Fab'-fragments to which at one of the -SH-groups in the hinge region a linker is bound. To a smaller extend also multiply labeled Fab'-fragments are present (cf. peaks III, II, and I, respectively, in Fig. 10).

## Example 5: Production of a second novel Fab'-BPRu(UE)$_{25}$-conjugate

### 1. F(ab')$_2$-fragments were prepared from an anti HIV p24 monoclonal

Fragmentation is essentially performed as described in Example 4. The F(ab')$_2$-solution is dialyzed against 50 mM potassium phosphate, 150 mM sodium chloride buffer of pH 7.5. To an F(ab')$_2$ - solution containing 5 mg/ml F(ab')$_2$ -fragments cysteamine in a final concentration of 25 mM is added. The solution is incubated for 60 minutes at 37 °C. Reaction is stopped via buffer change using a PD10-colum buffer to pH 6.0 with a 50 mM sodium phosphate, 150 mM sodium chloride buffer.

### 2. Ruthenylation of F(ab')$_2$-fragment

The linker BPRu-(UE)$_{25}$-MH is dissolved in DMSO in a concentration of 25 mg/ml. The molar ratio of F(ab')$_2$ -fragments to BPRu-(UE)$_{25}$-MH linker (maker) is set at 1 to 2. The reaction mixture is incubated for 90 minutes at 25 °C in a 50 mM potassium phosphate, 150 mM sodium chloride solution (pH 7.1). The reaction is stopped by addition of 2 mM cysteine and incubation of the solution for 30 minutes at 25 °C. N-Methyl-maleinimid is added at 5 mM and incubated for 60 minutes at 55 °C.

### 3. Purification

The crude conjugate was concentrated about 3-fold using a CENTRICON 10 and purified by means of a Superdex 200 HR® column buffered to pH 7.5 with 50 mM potassium phosphate, 150 mM sodium chloride. As can be seen from Figure 11, the crude mixture has been found to contain non-conjugated Fab'-fragment, non-conjugated linker (marker) as well as conjugates of uniform stoichiometries of 1:1, 1:2 and 1:3 (cf. peaks 5 and 4, 3, 2, and 1, respectively of Fig. 11).

## Example 6: Ruthenylation of F(ab')$_2$-fragments

### 1. Description of the procedure

### 1.1. Production of F(ab')$_2$ fragments of IgG

The same monoclonal antibody as known from example 5 is used for production of F(ab')$_2$-fragments. Digestion of IgG by pepsin is performed as described in Example 5. After quantitative cleavage pepsin is inactivated by increasing the pH and by adding pepstatin. F(ab')$_2$-fragments are purified using Superdex 200 HR® column chromatography.

### 1.2. Synthesis of F(ab')$_2$-BPRu-(UE)$_{25}$-DSS

Conjugation is performed by coupling the active group of BPRu-(UE)$_{25}$-DSS to amino groups of the F(ab')$_2$-fragment. Dependent on the initial concentration of reagents as well as to the stoichiometry chosen between the F(ab')$_2$-fragments and the linker (marker) molecules a crude conjugate is obtained with a higher or lower average labeling index. With other words, a random Poisson distribution of Fab'-molecules is obtained which carry varying amounts of linker structures.

### 1.3. Purification of crude conjugates

The crude conjugate is concentrated and purified using routine molecular sieve chromatography. Due to the unusual high apparent molecular weight, as contributed by each individual linker molecule, the coupling products of different stoichiometries can be separated from one another.

### 2. Procedure

### 2.1. Cleavage of IgG to F(ab')$_2$

Cleavage of Mab<p24>M-6D9-IgG to Mab<p24>M-6D9-F(ab')$_2$. Lyophylised IgG of a monoclonal antibody against p24 of HIV is reconstituted with H$_2$O to a final concentration 20 mg/ml. Cleavage is performed as described in Example 5.

### 2.2. Purification of F(ab')$_2$-fragments by Superdex 200 HR® chromatography.

The F(ab')$_2$-fragment produced as described are purified using the following buffers and conditions:

| Column material | Superdex 200 HR® |
|---|---|
| Buffer | 10 mM sodium phosphate, buffer adjusted to pH 7.5 30 mM sodium chloride |
| Fluoride | 10 ml/cm$^2$/h |
| Sample volume | 1.5 % of that volume of the column |
| Concentration of sample | 20 mg/ml |
| Monitoring of eluate photometer | 280 nm |

The fractions containing F(ab')$_2$-fragments are pooled concentrated and re-chromatographed under identical conditions.

### 2.3. Labeling of F(ab')$_2$-fragments with BPRu-(UE)$_{25}$-DSS

BPRu-(UE)$_{25}$-DSS is dissolved in DMSO at 5mg/ml. Coupling stoichiometry is chosen at 1:5 (mol/mol) = F(ab')$_2$-fragments : linker
The reaction mixture is incubated for 1 h at room temperature. Reaction is stopped by addition of lysine to a final concentration of 10 mM.

### 2.4. Coupling of F(ab')$_2$-fragments with BPRu-UEEK-DSS

The linker (label) BPRu-UEEK-DSS is dissolved in DMSO at a concentration of 5 mg/ml. The stoichiometry chosen for coupling is

$$1:5 \text{ (mol/mol)} = \text{F(ab')}_2\text{-fragments: BPRu-UEEK-DSS.}$$

The reaction mixture is incubated for 1 h at room temperature. Reaction is stopped by addition of lysine to a final concentration of 10 mM.

### 2.5. Purification of crude coupling products

Purification is performed by Superdex 200 HR® chromatography. Chromatography of both F(ab')$_2$-conjugates is performed in a 100 mM Hepes-buffer adjusted to pH 7.5, containing 1 mM EDTA and 5 % DMSO. The flow rate is set to 5 ml/cm$^2$/h. Detection is performed at 280 nm. Sample volume is 1.5 % of column volume.
As it is obvious from Figures 12 and 13 only the coupling products produced with the strongly negatively charged linker hy-BPRu-(UE)$_{25}$-DSS can be separated into fractions containing coupling products of uniform stoichiometry. The pooled fraction containing the majority of coupling products with 1:1 stoichiometry (ca. 107 min to 125 min; peak at 111 min) has been re-chromatographed once and purity as well as molecular weight been confirmed by MALDI-TOF MS.

### Example 7: Immunoassay using different antibody-linker conjugates

A double-antigen-test which is used for example in the detection of specific antibodies against HIV has been used as model system. The sample (suspected to contain antibodies against HIV) is incubated with a biotinylated antigen and a digoxigenin-labeled antigen in the presence of a streptavidin-coated solid phase and an BPRu-labeled antibody against digoxin. In the presence of anti-HIV-antibodies in a sample a detection complex is formed comprising the streptavidin-coated solid phase the biotinylated antigen the antibody to be detected, the digoxinylated antigen and the ruthenium-labeled antibody reactive with digoxin. The detection of anti-HIV-antibodies is performed by measuring the electrochemiluminescence-signal bound to the solid phase according to standard electrochemiluminescence procedures.
In this model system an HIV peptide from the gp41-reagion of HIV 1 has been used. Details relating to this peptide and labeling of this peptide are described in WO 96/03423.
All components of the system have been kept constant and only the detection system, i.e., the ruthenium-labeled anti-digoxin reagent has been varied. Both antigens have been used in a concentration of 20 ng/ml.

Table 5:

| Absolute counts measured | | | | |
|---|---|---|---|---|
| experiment [counts] | conjugate E | conjugate F | conjugate G | conjugate H |
| negative sample | 488 | 895 | 394 | 282 |
| Positive sample 1 | 65108 | 74993 | 59379 | 68469 |
| Positive sample 2 | 119557 | 138898 | 113507 | 134360 |

Table 6:

| Reaction normalized to negative sample | | | | |
|---|---|---|---|---|
| experiment pos./neg. sera | conjugate E | conjugate F | conjugate G | conjugate H |
| negative sample | 1,0 | 1,0 | 1,0 | 1,0 |
| positive sample 1 | 133,4 | 83,8 | 150,7 | 242,8 |
| positive sample 2 | 245,0 | 155,2 | 288,1 | 476,5 |

For all conjugates listed in Tables 5 and 6 a Fab'-fragment of an anti-DIG antibody has been used. Conjugates

**EP 1 245 956 A1**

E and F have been produced according to EP 720 614 using molar ratios of 1:2 and 1:6 (Fab' to linker), respectively. Conjugates G and H contain the purified 1:1 coupling products produced according to the present invention comprising the linker (label) structures shown in Figures 5 and 3, respectively.

The novel 1:1 conjugate allow for a better separation of positive sample from negative samples. This is most easily seen from table 6 where the signals obtained for the positive sere are normalized to the negative serum.

### List of References

Aslam M., Dent A., Bioconjugation (1998) Mcmillan Reference Ltd., London, p 216 to 363
Engvall, E. and Perlman, P., Immunochemistry 8 (1971) 871-4
Methods in Enzymology, Colowick S.P., Caplan N.O., Eds., Academic Press
Tijssen (1992) Elsevier Science Publishers B.V.
van Weemen, B.K. and Schuures A.H.W.M. (1971), FEBS letters 15, 232
S.-S. Wang, J. Am. Chem. Soc. 95 (1973) 1328
B.W. Bycroft et al. (1993), J. Chem. Soc., Chem. Commun., 778
A. Merzouk et al., (1992) Tetrahedron Lett. 33, 477
US 5,958,783
WO 96/03423
WO 96/03651
WO 96/03657
WO 96/03652

### Claims

1. Process for the production of a conjugate comprising at least one biomolecule-linker product of uniform stoichiometry in a pre-selected amount, said conjugate consisting of a biomolecule of a molecular weight between 5 kD and 500 kD and a hydrophilic linker molecule, said linker having a molecular weight between 1 kD and 15 kD, between 4 and 60 charged residues, **characterized in that**

   a) the biomolecule and the linker molecule are covalent coupled to each other,

   b) the different biomolecule-linker products of uniform stoichiometry are fractionated by chromatography, and

   c) the fractions containing a coupling product of uniform stoichiometry are collected.

2. The process according to claim 1, further **characterized in that** said hydrophilic linker comprises between 4 and 60 negatively charged residues.

3. The process according to claim 1, further **characterized in that** said hydrophilic linker comprises between 4 and 60 positively charged residues.

4. The process according to any of claims 1 to 3, further **characterized in that** the hydrophilic linker comprises 6 to 50 charged residues.

5. The process according to any of claims 1 to 4, further **characterized in that** said linker comprises a peptidic backbone.

6. The process according to any of claims 1 to 5, further **characterized in that** said biomolecule is a polypeptide.

7. The process according to any of claims 1 to 6, further **characterized in that** said chromatographic separation is based on differences in apparent molecular weight of said biomolecule linker products .

8. The process according to any of claims 1 or 7 further **characterized in that**, said hydrophilic linker has an apparent molecular weight of > 20 % and < 500 % of the apparent molecular weight of said biomolecule.

9.  The process according to any of claims 1 to 6, further **characterized in that** said chromatographic separation is based on differences in charge of said biomolecule-linker products.

10. Conjugate as produced according to any of claims 1 to 9.

11. Composition of reagents comprising buffer components, a stabilizing reagent, and a conjugate produced according to any of claims 1 to 9.

12. Test kit for biochemical or immunological detection of an analyte in a sample said kit comprising appropriate buffers and reagents and at least one composition of reagents according to claim 11.

13. Use of a conjugate according to any of claims 1 to 9 or a composition of reagents according to claim 11 in an immunoassay.

Figure 1: Schematic for BSA and its conjugation to hy-BPRu

Figure 2: Superdex 200 HR® chromatograph of a BSA-hy-BPRu-conjugate

Figure 3: Schematic of BPRu-(UE)$_{25}$ K and BPRu -(UE)$_{50}$ K

BPRu-(UE)$_{25}$K
BPRu-(UE)$_{50}$K

n = 25, 50

Figure 4: Branched linker with short backbone chain

Ac-EUEUEU
BPRu-UKKUEUEK
Ac-EUEUEU

Figure 5: Branched linker with long backbone chain

Ac-EUEUEUEU

BPRu-UKK(UE)25K

Ac-EUEUEUEU

Figure 6: Schematic of a branched linker without charged residues

Ac-QUQUQU

BPRu-UKKUQUQK —— CONH$_2$

Ac-QUQUQU

Figure 7: Schematic of biotinylated linker molecules

Bi-(UE)$_{25}$-K-MH
Bi-(UE)$_{25}$-K-DSS

Figure 8: Schematic of a multiply branched linker with two marker groups

Ac-EUEUEUEU

BPRu-UKKUE

Ac-EUEUEUEU

KUEK

Ac-EUEUEUEU

DSS

BPRu-UKKUE

Ac-EUEUEUEU

## Figure 9: Superdex 200 HR® chromatography of various linker molecules

Figure 10: Mono Q® chromatograph of a crude BSA-Ru(SK)$_4$-MH conjugate

Figure 11: Source 15 Q® chromatograph of a crude BSA-Ru(UE)$_{25}$-MH conjugate

Figure 12: Superdex 200 HR® 10/30 chromatograph of a first Fab'-Ru(UE)$_{25}$-MH coupling product

Figure 13: Superdex 200 HR® 10/30 chromatograph of a second Fab'-Ru(UE)$_{25}$ coupling product.

Figure 14: Purification of F(ab')$_2$-BPRu-(UE)$_{25}$-DSS coupling products

F(ab')$^2$-BPRu-(UE)$_{25}$-DSS
Free Linker     :     132.21 min
(label)
1 : 1 conjugate :     111.14 min
1 : 2 conjugate :     103.20 min

Figure 15: Purification of F(ab')$_2$-BPRu-UEEK-DSS coupling products

F(ab')$_2$-BPRu-UEEK-DSS
Free label    :    189.70 min
Product peak :    135.04 min

**European Patent Office**

## EUROPEAN SEARCH REPORT

**Application Number**

EP 01 10 7491

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X,D | US 5 958 783 A (OFENLOCH-HAEHNLE BEATUS ET AL) 28 September 1999 (1999-09-28) <br> * column 2, line 49 – column 3, line 25 * <br> * column 6, line 63 – column 7, line 64 * <br> * examples 2-4 * <br> * column 11, line 16,17 * <br> * claims 1,12-15,20-25 * | 1-13 | G01N33/531 <br> G01N33/53 <br> G01N33/68 |
| X,D | DE 44 30 972 A (BOEHRINGER MANNHEIM GMBH) 1 February 1996 (1996-02-01) <br> * page 4, line 55 – line 59 * <br> * examples 2-4 * | 1-8, 10-13 | |
| X | US 6 120 768 A (GOVINDAN SERENGULAM V ET AL) 19 September 2000 (2000-09-19) <br> * claims 1-34,40 * <br> * examples 1A,1B * | 1-10 | |
| X | US 5 047 324 A (FREDRICKSON ROBERT A) 10 September 1991 (1991-09-10) <br> * column 2, line 18 * <br> * column 2, line 27 – line 34 * <br> * column 2, line 35 – column 3, line 25 * | 1-8,10, 13 | |

**TECHNICAL FIELDS SEARCHED (Int.Cl.7)**

G01N

The present search report has been drawn up for all claims

| Place of search | Date of completion of the search | Examiner |
|---|---|---|
| MUNICH | 24 August 2001 | Stricker, J-E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**EP 1 245 956 A1**

## ANNEX TO THE EUROPEAN SEARCH REPORT
## ON EUROPEAN PATENT APPLICATION NO.

EP 01 10 7491

This annex lists the patent family members relating to the patent documents cited in the above-mentioned European search report.
The members are as contained in the European Patent Office EDP file on
The European Patent Office is in no way liable for these particulars which are merely given for the purpose of information.

24-08-2001

| Patent document cited in search report | | Publication date | Patent family member(s) | | Publication date |
|---|---|---|---|---|---|
| US 5958783 | A | 28-09-1999 | DE | 4430998 A | 01-02-1996 |
| | | | DE | 4439347 A | 01-02-1996 |
| | | | DE | 4439345 A | 01-02-1996 |
| | | | AT | 187732 T | 15-01-2000 |
| | | | AT | 193294 T | 15-06-2000 |
| | | | AU | 682278 B | 25-09-1997 |
| | | | AU | 3164995 A | 22-02-1996 |
| | | | AU | 689626 B | 02-04-1998 |
| | | | AU | 3165095 A | 22-02-1996 |
| | | | AU | 688953 B | 19-03-1998 |
| | | | AU | 3220495 A | 22-02-1996 |
| | | | AU | 690315 B | 23-04-1998 |
| | | | AU | 3220595 A | 22-02-1996 |
| | | | AU | 684992 B | 08-01-1998 |
| | | | AU | 3220695 A | 22-02-1996 |
| | | | CA | 2195648 A | 08-02-1996 |
| | | | CA | 2195752 A | 08-02-1996 |
| | | | CA | 2195753 A | 08-02-1996 |
| | | | CN | 1130910 A | 11-09-1996 |
| | | | CN | 1134154 A,B | 23-10-1996 |
| | | | CN | 1152923 A | 25-06-1997 |
| | | | CN | 1157655 A | 20-08-1997 |
| | | | DE | 4430972 A | 01-02-1996 |
| | | | DE | 4430973 A | 01-02-1996 |
| | | | DE | 4439346 A | 01-02-1996 |
| | | | DE | 59507438 D | 20-01-2000 |
| | | | DE | 59508391 D | 29-06-2000 |
| | | | WO | 9603650 A | 08-02-1996 |
| | | | WO | 9603651 A | 08-02-1996 |
| | | | WO | 9603652 A | 08-02-1996 |
| | | | WO | 9603409 A | 08-02-1996 |
| | | | WO | 9603423 A | 08-02-1996 |
| | | | WO | 9603410 A | 08-02-1996 |
| | | | EP | 0772616 A | 14-05-1997 |
| | | | EP | 0774119 A | 21-05-1997 |
| | | | EP | 0774120 A | 21-05-1997 |
| | | | EP | 0772774 A | 14-05-1997 |
| | | | EP | 0720614 A | 10-07-1996 |
| | | | EP | 0723551 A | 31-07-1996 |
| | | | ES | 2143059 T | 01-05-2000 |
| | | | ES | 2148540 T | 16-10-2000 |
| | | | FI | 961349 A | 25-03-1996 |
| | | | FI | 961350 A | 25-03-1996 |
| | | | FI | 970299 A | 24-01-1997 |
| | | | FI | 970300 A | 24-01-1997 |
| | | | FI | 970301 A | 24-03-1997 |

EPO FORM P0459

For more details about this annex : see Official Journal of the European Patent Office, No. 12/82

33

ANNEX TO THE EUROPEAN SEARCH REPORT
ON EUROPEAN PATENT APPLICATION NO.                    EP 01 10 7491

This annex lists the patent family members relating to the patent documents cited in the above-mentioned European search report.
The members are as contained in the European Patent Office EDP file on
The European Patent Office is in no way liable for these particulars which are merely given for the purpose of information.

24-08-2001

| Patent document cited in search report | Publication date | Patent family member(s) | Publication date |
|---|---|---|---|
| US 5958783 A | | JP 9508473 T | 26-08-1997 |
| | | JP 10506708 T | 30-06-1998 |
| | | JP 10504539 T | 06-05-1998 |
| | | JP 2771900 B | 02-07-1998 |
| DE 4430972 A | 01-02-1996 | AU 688953 B | 19-03-1998 |
| | | AU 3220495 A | 22-02-1996 |
| | | CA 2195753 A | 08-02-1996 |
| | | CN 1157655 A | 20-08-1997 |
| | | WO 9603652 A | 08-02-1996 |
| | | EP 0772774 A | 14-05-1997 |
| | | FI 970301 A | 24-03-1997 |
| | | JP 10504539 T | 06-05-1998 |
| | | KR 240514 B | 15-01-2000 |
| | | NO 970292 A | 13-03-1997 |
| | | NZ 291153 A | 28-01-1999 |
| | | AT 187732 T | 15-01-2000 |
| | | AT 193294 T | 15-06-2000 |
| | | AU 682278 B | 25-09-1997 |
| | | AU 3164995 A | 22-02-1996 |
| | | AU 689626 B | 02-04-1998 |
| | | AU 3165095 A | 22-02-1996 |
| | | AU 690315 B | 23-04-1998 |
| | | AU 3220595 A | 22-02-1996 |
| | | AU 684992 B | 08-01-1998 |
| | | AU 3220695 A | 22-02-1996 |
| | | CA 2195648 A | 08-02-1996 |
| | | CA 2195752 A | 08-02-1996 |
| | | CN 1130910 A | 11-09-1996 |
| | | CN 1134154 A,B | 23-10-1996 |
| | | CN 1152923 A | 25-06-1997 |
| | | DE 4430973 A | 01-02-1996 |
| | | DE 4430998 A | 01-02-1996 |
| | | DE 4439345 A | 01-02-1996 |
| | | DE 4439346 A | 01-02-1996 |
| | | DE 4439347 A | 01-02-1996 |
| | | DE 59507438 D | 20-01-2000 |
| | | DE 59508391 D | 29-06-2000 |
| | | WO 9603650 A | 08-02-1996 |
| | | WO 9603651 A | 08-02-1996 |
| | | WO 9603409 A | 08-02-1996 |
| | | WO 9603423 A | 08-02-1996 |
| | | WO 9603410 A | 08-02-1996 |
| | | EP 0772616 A | 14-05-1997 |
| | | EP 0774119 A | 21-05-1997 |
| | | EP 0774120 A | 21-05-1997 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 01 10 7491

This annex lists the patent family members relating to the patent documents cited in the above-mentioned European search report.
The members are as contained in the European Patent Office EDP file on
The European Patent Office is in no way liable for these particulars which are merely given for the purpose of information.

24-08-2001

| Patent document cited in search report | Publication date | Patent family member(s) | | Publication date |
|---|---|---|---|---|
| DE 4430972 A | | EP | 0720614 A | 10-07-1996 |
| | | EP | 0723551 A | 31-07-1996 |
| | | ES | 2143059 T | 01-05-2000 |
| | | ES | 2148540 T | 16-10-2000 |
| | | FI | 961349 A | 25-03-1996 |
| | | FI | 961350 A | 25-03-1996 |
| | | FI | 970299 A | 24-01-1997 |
| | | FI | 970300 A | 24-01-1997 |
| US 6120768 A | 19-09-2000 | US | 5736119 A | 07-04-1998 |
| | | AU | 1825899 A | 05-07-1999 |
| | | WO | 9930745 A | 24-06-1999 |
| | | AU | 699216 B | 26-11-1998 |
| | | AU | 6038496 A | 30-12-1996 |
| | | CA | 2223261 A | 19-12-1996 |
| | | EP | 0837696 A | 29-04-1998 |
| | | JP | 11507046 T | 22-06-1999 |
| | | WO | 9640245 A | 19-12-1996 |
| | | US | 6228362 B | 08-05-2001 |
| | | US | 5846741 A | 08-12-1998 |
| | | US | 5965131 A | 12-10-1999 |
| | | US | 5958408 A | 28-09-1999 |
| | | AU | 4047497 A | 20-02-1998 |
| | | WO | 9804293 A | 05-02-1998 |
| | | US | 5922302 A | 13-07-1999 |
| | | AU | 683893 B | 27-11-1997 |
| | | AU | 6908994 A | 12-12-1994 |
| | | CA | 2163107 A | 24-11-1994 |
| | | EP | 0700304 A | 13-03-1996 |
| | | JP | 8510250 T | 29-10-1996 |
| | | KR | 220864 B | 15-09-1999 |
| | | WO | 9426297 A | 24-11-1994 |
| US 5047324 A | 10-09-1991 | AU | 598323 B | 21-06-1990 |
| | | AU | 8200987 A | 09-06-1988 |
| | | CA | 1302251 A | 02-06-1992 |
| | | DK | 643587 A | 10-05-1988 |
| | | EP | 0270930 A | 15-06-1988 |
| | | IL | 84108 A | 29-03-1992 |
| | | NO | 874938 A | 10-06-1988 |
| | | IE | 333287 L | 09-06-1988 |
| | | JP | 63198871 A | 17-08-1988 |
| | | ZA | 8708431 A | 05-05-1988 |

EPO FORM P0459

For more details about this annex : see Official Journal of the European Patent Office, No. 12/82